# FASCICULE DE BREVET EUROPEEN

(11) **EP 2 087 121 B1**
(45) Date de publication et mention de la délivrance du brevet: **14.06.2017**
(21) Numéro de dépôt: 07821735.3
(22) Date de dépôt: 23.10.2007
(51) Int. Cl.: C12N 15/82, C12N 15/29, C12N 15/01, C12N 15/87

(54) **MAIS PRESENTANT UNE TOLERANCE ACCRUE AUX MALADIES FONGIQUES**
MAIS MIT VERBESSERTER TOLERANZ GEGENÜBER PILZKRANKHEITSERREGERN
MAIZE WITH ENHANCED TOLERANCE TO FUNGAL PATHOGENS

(30) Priorité: 24.10.2006 FR 0609295
(43) Date de publication de la demande: 12.08.2009
(73) Titulaire: Biogemma, 75001 Paris (FR)
(72) Inventeur: TATOUT, Christophe, 42110 Salt En Donzy (FR); GREZES-BESSET, Bruno, 31770 Colomiers (FR); GEORGE, Pierre, 31530 Levignac (FR)
(74) Mandataire: Flesselles, Bruno F.G.
(86) Numéro de dépôt international: PCT/EP2007/061372
(87) Numéro de publication internationale: WO 2008/049848

(56) Documents cités:
- EP-A- 0 516 958
- EP-A- 1 000 543
- WO-A-99/24561
- WO-A-2006/035045
- FOFANA BOURLAYE ET AL: "Suppression of induced resistance in cucumber through disruption of the flavonoid pathway" PHYTOPATHOLOGY, vol. 95, no. 1, janvier 2005 (2005-01), pages 114-123, XP009081963 ISSN: 0031-949X
- LAMB C J ET AL: "Emerging strategies for enhancing crop resistance to microbial pathogens" BIO/TECHNOLOGY 1992 UNITED STATES, vol. 10, no. 11, 1992, pages 1436-1445, XP002032892 ISSN: 0733-222X
- BILY A C ET AL: "Dehydrodimers of ferulic acid in maize grain pericarp and aleurone: Resistance factors to Fusarium graminearum." PHYTOPATHOLOGY, vol. 93, no. 6, juin 2003 (2003-06), pages 712-719, XP001249375 ISSN: 0031-949X cité dans la demande
- GOUJON THOMAS ET AL: "Down-regulation of the AtCCR1 gene in Arabidopsis thaliana: effects on phenotype, lignins and cell wall degradability." PLANTA JUN 2003, vol. 217, no. 2, juin 2003 (2003-06), pages 218-228, XP002429128 ISSN: 0032-0935
- BOUDET A M ET AL: "LIGIN GENETIC ENGINEERING" MOLECULAR BREEDING, KLUWER ACADEMIC PUBLISHERS, DORDRECHT, NL, vol. 2, janvier 1996 (1996-01), pages 25-39, XP002025844 ISSN: 1380-3743

## Description

La présente invention se rapporte au domaine de l'amélioration du maïs, en particulier de la tolérance du maïs aux pathogènes fongiques et notamment à la fusariose par l'altération qualitative et/ou quantitative de la voie de biosynthèse de la lignine.

Les maïs sont soumis aux attaques de nombreux pathogènes. Parmi ceux-ci, on peut citer les virus, bactéries, mais aussi les pathogènes fongiques, responsables de nombreuses maladies, et parfois de la présence de mycotoxines.

Ainsi, le maïs peut être attaqué par des champignons responsables de fusarioses (due aux *Fusarium,* dont *F. roseum*, *F. gramninearum*, *F. liseola*, *F. monoliforme*), de charbon (commun ou des inflorescences, du à *Ustilago zeae*, *Ustilago maydis*), d'anthracnose (*Colletotrichum graminicola*), de kabatiellose, d'helminthosporiose (*Helminthosporium turcicum*), de rouille (*Puccinia maydis*), de mildiou. D'une façon générale, les attaques fongiques sont responsables de dessèchement et/ou de pourriture des plantes, qui sont localisées différemment selon le pathogène.

Les champignons du genre *Fusarium* sont responsables de la fusariose, on peut citer les espèces *F. graminearum*, et *F. moniliforme* pathogènes du maïs et dont l'importance varie suivant les conditions climatiques et la précocité des variétés de maïs. On peut distinguer la fusariose de l'épi de la fusariose de la tige, les processus infectieux étant très différents. Certains agents pathogènes sont cependant communs aux deux types de fusariose. Plusieurs modes de contamination de la plante par le champignon sont connus, comme la pénétration du mycélium infectieux dans la plante par des lésions imputables aux insectes et aux oiseaux, ou bien la pénétration directe au niveau des soies de l'épi aboutissant à l'infection des grains. Dans le cas de la fusariose de la tige, la contamination peut également avoir lieu via les semences ou plus rarement les racines.

La fusariose de l'épi qui entraîne une destruction des grains diminue le rendement des cultures de maïs. Les agents pathogènes en cause sont par ailleurs très dommageables car ils accumulent dans les grains détruits ou non différentes mycotoxines (Zéaralenone, Deoxynivalénol, Fumonisines), qui présentent des niveaux de toxicité variables suivant les espèces animales et sont difficiles à éliminer.

Les traitements fongicides sont difficiles d'utilisation et n'ont qu'un effet limité à l'encontre des *Fusarium.* La meilleure façon de combattre la fusariose de l'épi est l'utilisation de la résistance génétique. Peu d'hybrides possèdent actuellement une telle résistance, quand elle existe il s'agit d'un résistance partielle qui reste modérée.

Il apparaît donc important d'identifier des méthodes pour augmenter la tolérance aux maladies fongiques chez le maïs.

L'approche utilisée par les inventeurs a consisté à modifier l'expression des gènes de la voie des phénylpropanoïdes, et plus particulièrement la voie de biosynthèse de la lignine. Cette voie des phénylpropanoïdes conduit, à partir de la phénylalanine, à la synthèse d'une grande variété de substances telles que les anthocyanes, les isoflavonoïdes, les stilbènes, des ester d'acides hydroxycinnamiques, ou encore à la lignine. La lignine assure rigidité aux parois cellulaires et imperméabilité aux tissus conducteurs. Ainsi, des modifications dans la voie des phénylpropanoïdes ont des conséquences sur la synthèse de lignine.

Un lien entre lignine et la tolérance aux pathogènes est communément suggéré, la lignine ayant notamment un rôle de barrière physique envers les pathogènes extérieurs, limitant la pénétration des pathogènes. Ainsi, lors de réactions incompatibles il y a accumulation de lignine au site de la HR (Hypersensitive Response). Toutefois, aucune démonstration directe de ce rôle n'a été décrite dans l'art antérieur.

Cependant, de nombreuses équipes ont travaillé sur l'inhibition d'enzymes de la voie de biosynthèse de la lignine afin de modifier la quantité ou la qualité de lignines du maïs, en particulier pour obtenir des plantes présentant une digestibilité accrue.

La modification de la teneur et composition des plantes en lignines peut s'obtenir en surexprimant ou sous exprimant un ou plusieurs gènes de la voie de biosynthèse des lignines (Anterola et Lewis, 2002, Phytochemistry 61, 221-294). De tels travaux sont décrits notamment dans les demandes de brevets WO 9924561, EP0516958, WO9305160, WO9712982 qui exposent les différentes stratégies imaginées.

La Cinnamoyl Coenzyme-A Réductase (CCR) intervient dans la voie de biosynthèse des lignines pour convertir les p-coumaroyl CoA, feruloyl CoA et sinapoyl CoA en aldéhydes. L'existence des ces trois substrats laisse penser que soit il existe plusieurs isoformes de CCR soit la ou les CCR sont capables de catalyser les réactions à partir d'une grande variété de substrats, à moins qu'il n'existe des isoformes de CCR différentes selon le type cellulaire. Aucune de ces hypothèses n'a été clairement prouvée à ce jour.

Chez le maïs, à ce jour, deux isoformes de la CCR ont été isolées (Pichon et al., 1998, Plant Mol Biol, 38, 671-676). Il semblerait que seule la CCR1 (SEQ ID N° 1) soit impliquée dans la voie de biosynthèse des lignines.

La Caffeoyl Coenzyme-A O-Méthyl Transférase (CCoAOMT) est une enzyme importante de la voie de biosynthèse des monolignols et plus particulièrement des sous-unités G. La CCoAOMT semble intervenir au cours de plusieurs étapes de la voie de biosynthèse des lignines. Ainsi, elle serait impliquée dans une voie alternative de méthylation de la biosynthèse des lignines chez les zinnia, et la voie de méthylation médiée par la CCoAOMT serait probablement une des voies générales de la biosynthèse des lignines pendant la croissance et le développement de la plante.

Chez le maïs, deux gènes codent pour la CCoAOMT, le gène CCoAOMT1 et le gène CCoAOMT2. Le gène CCoAOMT2 est situé sur le chromosome 9, le gène CCoAOMT1 se situant sur le chromosome 6.

Les demandes de brevet WO9910498 et WO0134817 concernent entre autres, le gène de la CCoAOMT1 de maïs.

La cinnamate 4-hydroxylase (C4H) existe au moins sous deux formes, en fonction des espèces considérées (C4H-1 et C4H-2). La forme C4H-1 est impliquée dans la lignification et le métabolisme des phénylpropanoïdes, alors que le rôle de la C4H-2 est encore peu clair. Des expériences de dérégulation de la C4H-1 laisse entendre que ce gène est une étape limitante dans la formation de lignines, la dérégulation résultant apparemment en une diminution progressive et une réduction quantitative (rapport S/G) de la lignine.

La 4-Coumarate:CoA ligase (4CL) est généralement présente sous la forme d'une famille multigénique dont les isoformes ont des spécificités de substrats différentes, de même que leurs profils d'expression spatiaux-temporels. Son rôle dans la lignification a été étudié notamment dans le tabac et chez *Arabidopsis.*

Bily et al., 2003 (Phytopathology 93:712-719) ont travaillé sur la relation entre des composés de la paroi et la résistance du maïs à des *Fusarium* chez différentes variétés de maïs. Ils ont observé chez les variétés présentant une résistance plus grande aux *Fusarium* la présence d'une quantité plus importante de dimères d'acide férulique, par contre aucune corrélation n'est démontrée entre les variétés résistantes et la présence de monomères d'acide férulique ou d'acide p-coumarique. Ces travaux mettent en évidence l'importance d'éléments associés à la lignine dans la tolérance aux pathogènes, mais ne permettent pas de faire d'hypothèse sur une manipulation possible de la voie de biosynthèse de la lignine pour augmenter la tolérance aux pathogènes fongiques.

Ainsi, s'il est donc bien connu que l'on peut manipuler les gènes autologues impliqués dans la voie de biosynthèse de la lignine pour modifier la qualité et/ou la quantité de lignine, il n'est pas démontré qu'une telle manipulation puisse augmenter la tolérance aux pathogènes fongiques.

La présente invention apporte des faits expérimentaux inattendus, démontrant que la dérégulation (inhibition) d'enzymes de la voie de biosynthèse de la lignine chez le maïs augmente la tolérance dudit maïs aux pathogènes fongiques.

### Description :

La présente invention se rapporte ainsi à une méthode pour augmenter la tolérance à un pathogène fongique chez le maïs comprenant une étape consistant à altérer qualitativement et/ou quantitativement la synthèse de lignine, par l'inhibition totale ou partielle de l'expression d'au moins un gène impliqué dans la voie de biosynthèse de la lignine (ou des phénylpropanoïdes), la tolérance audit pathogène fongique étant augmentée par rapport à un maïs dans lequel ledit gène n'est pas inhibé et/ou la synthèse de lignine n'est pas altérée. La tolérance ou résistance aux maladies fongiques selon l'invention s'entend comme une retard d'apparition des symptômes après infection par le pathogène pour la plante selon l'invention par rapport à une plante témoin, et/ou ou à une intensité moindre des symptômes observés à une date donnée après infection (notamment la surface de feuilles attaquée). Le gène code pour la cinnamoyl CoA reductase (CCR).

Par « altération quantitative de la synthèse de lignine », on entend désigner une diminution de la quantité de lignine dans le maïs modifié selon l'invention par rapport à un maïs normal (témoin non modifié selon l'invention), évalué, par exemple par mesure de la lignine de Klason ou de la lignine obtenue par détergent acid (acid détergent lignin) selon des méthodes bien connues dans l'art (voir par exemple Jung et al, J Agric Food Chem. 1999 May;47(5):2005-8 ; Jung et al, J Dairy Sci. 1997 Aug;80(8): 1622-8).
Par « altération qualitative de la synthèse de lignine », on entend désigner une modification dans la composition de la lignine de la plante modifiée selon l'invention par rapport à une plante témoin (non modifiée selon l'invention), par exemple une variation du rapport des sous unités S/G, ou une modification de la qualité d'acide férulique. Les méthodes d'analyse qualitative de la lignine sont également connues dans l'art. On peut notamment citer la RMN.
La présente demande décrit plusieurs gènes de la voie de biosynthèse de la lignine, et notamment les gènes codant pour les enzymes : cinnamoyl CoA reductase 1 (CCR1, EC 1.2.1.44, SEQ ID N° 1, SEQ ID N° 2 ; objet de l'invention), Caffeoyl Coenzyme A 3-O-methyltransferase (CCaOMT, SEQ ID N° 3 ; SEQ ID N° 4), cinnamate 4-hydroxylase (C4H, SEQ ID N° 5), 4-coumarate :CoA ligase (4CL, SEQ ID N° 6).
Il convient de noter que les séquences fournies dans la liste de séquences ne doivent être considérées qu'en tant qu'illustrations de ces allèles. Il est clair que l'homme du métier, en utilisant ces séquences, est capable d'isoler ces mêmes gènes pour d'autres variétés de maïs (autres allèles), notamment en isolant, dans le génome d'une autre variété, l'allèle considéré par PCR, ou Southern Blot, puis en le séquençant.

Dans le cadre de l'invention, le gène de la voie de biosynthèse de la lignine dont l'expression est inhibée est le gène CCR1, dont les séquences d'allèles représentatifs de maïs sont SEQ ID N° 1 et SEQ ID N° 2.

Ainsi, on met en oeuvre l'invention, en inhibant totalement ou partiellement le gène de la CCR1 représenté par SEQ ID N° 1 (ou tout autre allèle) chez le maïs.

L'inhibition du gène codant pour CCR1, impliqué dans la voie de biosynthèse de la lignine peut être obtenue par tout moyen connu dans l'art. Ainsi, on peut procéder à la mutation des gènes par insertion d'un élément transposable ou d'un ADN de transfert (T-DNA). On peut aussi effectuer une mutagenèse physique ou chimique, notamment par l'utilisation d'EMS, de rayons X ou d'ultraviolets.

Les plantes ainsi mutées sont criblées par exemple par PCR, en utilisant des amorces situées dans le gène cible. Toutefois, on peut aussi utiliser d'autres méthodes de criblage, comme les Southern Blots ou un criblage via la méthode AIMS décrite dans WO 99/27085 (pour détecter les insertions), en utilisant des sondes spécifiques des gènes cibles, ou les méthodes de détection de mutations ponctuelles ou de petites insertions/délétions utilisant des endonucléases particulières (Cel I, Endo I) telles que décrites dans WO 2006/010646.
Dans un autre mode de réalisation, l'inhibition est obtenue par transformation de la plante avec un vecteur contenant un construit sens ou anti-sens du gène cible. Ces deux méthodes sont connues pour permettre l'inhibition du gène cible. On utilise également la méthode de l'interférence d'ARN (RNAi) qui est particulièrement efficace pour l'extinction de gènes dans les plantes. Cette méthode est bien connue de l'homme du métier et consiste en la transformation de la plante avec un construit produisant, après transcription, un duplex double-brin d'ARN, dont l'un des brins est complémentaire à l'ARNm du gène cible.
L'invention permet ainsi d'obtenir un maïs présentant une tolérance à un pathogène fongique, en particulier du genre *Fusarium*, préférentiellement choisi parmi *F. graminearum*, *F. liseola*, *F. roseum* et *F. monoliforme.* Le maïs présente également une tolérance aux autres pathogènes fongiques cités plus avant et notamment aux pathogènes du genre *Helminthosporium*, en particulier *H*. *turcicum.*

Dans un mode de réalisation préférée, la présente invention est mise en oeuvre en utilisant un allèle favorable de la CCR1 (appelé Δ3318) de maïs, une insertion ayant été effectuée dans le premier intron du gène codant pour cette enzyme. La séquence de l'ARNm correspondant de la CCR1 de maïs est disponible sous le numéro X98083 de GenBank (SEQ ID N° 5), avec une partie codante des nucléotides 79 à 1194. Il est clair que ces séquences ne sont données qu'à titre d'exemples, et que l'homme du métier est à même d'identifier lui-même les séquences génomique et/ou d'ARNm de la CCR1 pour différentes variétés de maïs.

Ainsi, un autre allèle correspondant à la séquence de la CCR1 de maïs non mutée est représenté par l'accession Y13734 (GenBank).

Des graines de maïs possédant l'allèle Δ3318 ont été déposées au NCIMB Limited, 23 St Machar Drive, Aberdeen, Scotland, AB24 3RY, UK, le 23 juillet 2004, selon les dispositions du Traité de Budapest, sous le numéro NCIMB 41236. Ce maïs contenant l'allèle Δ3318 a été décrit dans WO 2006/035045, dont l'enseignement est inclus par référence.
L'invention peut être mise en oeuvre, pour le maïs, *via* une méthode permettant d'obtenir un maïs présentant une tolérance aux pathogènes fongiques, qui comprend l'étape d'introgression de l'allèle Δ3318 dans ledit maïs, ledit maïs contenant l'allèle Δ3318 présentant une tolérance accrue aux pathogènes fongiques par rapport à un maïs ne contenant pas ledit allèle.
L'introgression correspond à l'infiltration progressive de l'allèle Δ3318 d'un maïs le contenant dans un autre maïs ne le contenant pas initialement, par suite d'une hybridation interspécifique suivie de rétro-croisements en retour successifs avec le parent receveur (méthode de back-cross).
Ainsi, on peut réaliser l'introgression via les étapes consistant à :
(a) croiser une première lignée de maïs présentant l'allèle Δ3318 avec une seconde lignée de maïs ne présentant pas ledit allèle,
(b) effectuer un génotypage de la descendance obtenue et sélectionner les descendants présentant l'allèle Δ3318, et ayant le meilleur génome ratio pour ce qui est de ladite seconde lignée de maïs,
(c) effectuer un rétrocroisement desdits descendants avec ladite seconde lignée de maïs élite utilisable pour la production d'hybrides,
(d) répéter si nécessaire les étapes b) et c) jusqu'à obtenir une lignée isogénique dudit second maïs, présentant l'allèle Δ3318,
(e) de façon optionnelle, effectuer une auto-fécondation afin d'obtenir une plante homozygote pour l'allèle Δ3318.
Le génotypage de l'étape b) est effectué préférentiellement en utilisant des marqueurs moléculaires (marqueurs microsatellites par exemple), permettant de définir la part de chacun des deux parents dans la descendance. On sélectionne également, dans la descendance, les maïs qui possèdent le caractère génétique approprié pour ce qui est de l'allèle Δ3318, de façon classique, par les méthodes de biologie moléculaire (telles que PCR ou Southern Blot). On utilise préférentiellement la méthode et les amorces décrites dans WO 2006/035045.

De manière surprenante, il a été montré que la répétition des rétrocroisements entre les lignées sélectionnées à l'étape b) et la seconde lignée de maïs (second maïs) permet d'obtenir l'apparition d'un phénotype bien plus marqué au sein dudit second maïs. Ce résultat est tout à fait surprenant car on aurait pu s'attendre à observer une amélioration de la tolérance aux maladies fongiques, et notamment à la fusariose dès le premier croisement du maïs présentant l'allèle Δ3318 avec le second maïs.

L'invention peut également être complétée *via* une méthode dans laquelle on introgresse également un allèle du gène C4H, ledit allèle étant appelé D1938, dans le maïs dans lequel on introgresse l'allèle Δ3318. Des graines possédant l'allèle D1938 ont été déposées au NCIMB Limited, Ferguson Building, Craibstone Estate, Bucksburn, Aberdeen, Scotland, AB21 9YA, UK, le 15 octobre 2007, selon les dispositions du Traité de Budapest, sous le numéro NCIMB 41507.

De façon préférée, l'invention est mise en oeuvre sur des plantes « élites », c'est-à-dire notamment des plantes destinées à être commercialisées après inscription sur un catalogue de référence. Il est important de noter que le caractère « élite » d'une plante est défini en relation avec le territoire envisagé pour la commercialisation, ainsi que le(s) caractère(s) agronomique(s) recherché(s). Ainsi, certaines plantes sont particulièrement adaptées pour certaines régions, et seront ainsi considérées comme « élites » pour la culture dans ces régions, alors qu'elles ne le seront pas pour d'autres régions, par exemple dans lesquelles les conditions environnementales sont différentes.

Ainsi, une plante élite est une plante rassemblant le maximum de caractéristiques agronomiques nécessaires pour une pénétration économique du marché visé. Pour les marchés d'hybrides (par exemple le marché du maïs), l'évaluation du caractère élite des plantes s'effectue également par leur aptitude à la combinaison/production d'hybrides.

De façon préférée, l'invention est mise en oeuvre avec des maïs « élite ». L'homme du métier connaît bien la définition d'un maïs élite. Par maïs élite, on entend un maïs destiné à générer des hybrides destinés à être commercialisés par croisement avec un autre maïs élite. Un maïs élite est défini comme tel en relation avec le territoire envisagé pour la commercialisation, ainsi que le(s) caractère(s) agronomique(s) recherché(s) pour la descendance hybride. Il s'agit notamment d'un maïs pouvant être inscrit dans un catalogue de référence. Ainsi, selon que la descendance est destinée à l'alimentation humaine ou animale, on recherchera par exemple respectivement un rendement en grains, ou un rendement à l'hectare et une bonne digestibilité, lorsque l'on évalue la nature « élite du maïs ». Afin de déterminer le caractère élite d'un maïs, on compare des hybrides obtenus à partir de celui-ci aux hybrides commerciaux de référence (vendus pour le même objectif dans la même région), par des essais en champs, par relevé et mesures de caractères agronomiques appropriés à l'objectif recherché. Un maïs est défini comme élite si les résultats obtenus sur les paramètres étudiés pour un hybride obtenu par croisement dudit maïs sont supérieurs à 90 % aux résultats relevés pour les mêmes paramètres des hybrides de référence. Dans le cadre de la présente invention, on étudie, en plus des caractères agronomiques recherchés, la capacité de tolérance aux maladies fongiques, et en particulier à la fusariose.

Ainsi, un maïs élite est un maïs rassemblant le maximum de caractéristiques agronomiques nécessaires pour une pénétration économique du marché visé. Le marché du maïs étant aujourd'hui un marché d'hybrides, l'évaluation du caractère élite d'un maïs s'effectue également par l'aptitude dudit maïs à la combinaison/production d'hybrides.

Ainsi, la présente invention permet la préparation d'un maïs élite destiné notamment à la commercialisation d'hybrides, pour l'alimentation humaine, animale ou l'ensilage, présentant l'allèle Δ3318. Ce maïs élite est donc homozygote pour l'allèle Δ3318.

L'invention permet également la préparation et l'utilisation d'un maïs hybride obtenu par croisement de deux lignées parentes homozygotes, ledit maïs hybride présentant un allèle Δ3318. Ce maïs hybride peut être homozygote (si chaque parent homozygote présente l'allèle Δ3318) ou hétérozygote pour l'allèle Δ3318.
L'invention permet d'obtenir un maïs ou un grain de maïs, contenant un ou plusieurs transgènes en plus de l'allèle Δ3318. On peut citer des transgènes conférant une stérilité mâle, une fertilité mâle, la résistance à un herbicide (notamment le glyphosate, le glufosinate, l'imidazolinone, la sulfonylurée, la L-phosphinotricine, la triazine, le benzonitrile), la résistance aux insectes (notamment un transgène codant pour une toxine de Bacillus thuringiensis), la tolérance au stress hydrique. Ces maïs peuvent être obtenus en croisant un maïs contenant l'allèle Δ3318 avec un maïs contenant ledit transgène. La mise en oeuvre de rétrocroisements suivi d'une autofécondation permet d'obtenir un maïs élite homozygote pour l'allèle Δ3318 et le trangène. Toutefois, un maïs hybride contenant à la fois l'allèle Δ3318 et le transgène peut également être obtenue par la mise en oeuvre de l'invention.
L'invention mène donc à l'obtention d'un maïs ou un grain de maïs comprenant une inhibition ou une sous-expression de l'expression d'un gène de la voie de biosynthèse de la lignine, ainsi qu'un ou plusieurs transgènes, le gène codant pour CCR1.

### EXEMPLES

### Exemple 1 : Analyse phénotypiques de lignées de maïs mutants pour les gènes de la voie des phénylpropanoïdes

On dispose d'une plante mutante homozygote et d'un contrôle homozygote sauvage pour chaque événement d'insertion. Étant donné les stades avancés d'introgression de la mutation, on peut considérer que le mutant et le sauvage ne diffèrent que par la présence ou non de la mutation. L'expérimentation est réalisée selon le protocole suivant :
- 2 lieux
- 3 répétitions par lieu
- Inoculation artificielle de *Fusarium monoliforme*
- Notation des symptômes observés sur épis (note de 1 à 7). Il s'agit d'une notation visuelle d'intensité d'attaque sur épis : la note d'intensité d'attaque est calculée à partir du pourcentage de la surface de l'épi attaqué par le pathogène (Reid et al, Agriculture and Agri-Food Canada, Ottawa, Ont. Technical Bulletin 1996-5E. 40 pp). Les notes correspondent à : 1 = 0% attaqué ; 2 = 1-3% ; 3 = 4-10% ; 4 = 11-25% ; 5 = 26-50% ; 6 = 51-75% ; 7 = 76-100%.
- Eventuellement dosage des mycotoxines (fumonisines)

Dans les exemples ci-dessous, les répétitions « lieux » ont été converties en « répétitions simples » de façon à réaliser une analyse statistique simple. L'analyse statistique a été réalisée sur chaque mutant de façon à savoir s'il existe une différence entre mutant (M) et sauvage ou témoin (S).

Les résultats démontrent que l'insertion d'un transposon dans les gènes C4H, CCR1, 4CL2, CCoAOMT1, CCoAOMT2, impliqués dans la voie du métabolisme des phénylpropanoïdes et de la lignine augmente de façon plus ou moins importante la tolérance à une infection par *Fusarium monoliforme.* Cet effet est reproductible selon les lieux de culture, bien qu'il puisse être plus ou moins accentué en fonction des contraintes environnementales.

**Mutant B0682_C4H**

| Méthode: 95,0 % LSD | | | |
|---|---|---|---|
| **TYPE** | **Effectif** | **Moyennes** | **Ecarts-types** |
| M | 61 | 4,42623 | 0,22603 |
| S | 70 | 4,74471 | 0,189099 |

| **Contraste** | **Différence** | | **+/- limites** |
|---|---|---|---|
| M - S | -0,318485 | | 0,583536 |

| | | | |
|---|---|---|---|
| * indique une différence statistiquement significative. **Tendance Mutant plus résistant que sauvage.** | | | |

**Mutant D1938_C4H**

| Méthode: 95,0 % LSD | | | |
|---|---|---|---|
| **TYPE** | **Effectif** | **Moyennes** | **Ecarts-types** |
| M | 72 | 2,9163 | 0,124743 |
| S | 81 | 3,8593 | 0,117351 |

| **Contraste** | **Différence** | | +/- **limites** |
|---|---|---|---|
| M - S | *-0,942997 | | 0,338582 |

| | | | |
|---|---|---|---|
| * différence statistiquement significative. **Mutant plus résistant que sauvage ; dépendance environnementale.** | | | |

**Mutant Δ3318_CCR1**

| **TYPE** | **Effectif** | **Moyennes** | **Ecarts-types** |
|---|---|---|---|
| M | 103 | 3,2961 | 0,137621 |
| S | 93 | 4,69213 | 0,143085 |

| **Contraste** | **Différence** | | **+/- limites** |
|---|---|---|---|
| M - S | *-1,39603 | | 0,391682 |

| | | | |
|---|---|---|---|
| * différence statistiquement significative. **Mutant plus résistant que sauvage : effet net de 1,4 points en moyenne** | | | |

**Mutant I1888_4CL2**

| **TYPE** | **Effectif** | **Moyennes** | **Ecarts-types** |
|---|---|---|---|
| M | 95 | 4,5253 | 0,125475 |
| S | 57 | 4,5412 | 0,163631 |

| **Contraste** | **Différence** | | **+/- limites** |
|---|---|---|---|
| M - S | 0,0158996 | | 0,407671 |

| | | | |
|---|---|---|---|
| **Tendance mutant plus résistant, mais forte dépendance environnementale.** | | | |

**Mutant F0046_CCoAOMT1**

| **TYPE** | **Effectif** | **Moyennes** | **Ecarts-types** |
|---|---|---|---|
| M | 98 | 2,70723 | 0,120904 |
| S | 100 | 3,04586 | 0,119426 |

| **Contraste** | **Différence** | | **+/- limites** |
|---|---|---|---|
| M - S | *-0,338633 | | 0,335263 |

| | | | |
|---|---|---|---|
| * différence statistiquement significative. **Mutant plus résistant que sauvage, effet modéré.** | | | |

**Mutant G2092_CCoAOMT2**

| **TYPE** | **Effectif** | **Moyennes** | **Ecarts-types** |
|---|---|---|---|
| M | 81 | 3,07471 | 0,129118 |
| S | 103 | 3,71153 | 0,114828 |
| **Contraste** | **Différence** | | **+/- limites** |
| M - S | *-0,636821 | | 0,341066 |

| | | | |
|---|---|---|---|
| * différence statistiquement significative. **Mutant plus résistant que sauvage ; effet modéré** | | | |

### Analyses des mycotoxines

Un dosage de mycotoxines a été réalisé. On observe qu'il existe une bonne corrélation entre le niveau d'infection et l'accumulation de la fumonisine. Il a été remarqué que le taux de fumonisine observé est particulièrement élevé par rapport aux taux observés lors des infections naturelles. Le système d'infection artificielle dans le canal des soies n'est peut-être pas le plus adapté à l'étude de l'accumulation des mycotoxines.

### Exemple 2 : Réalisation de plantes transgéniques portant une cassette d'expression permettant l'inhibition d'un gène du métabolisme des phénylpropanoïdes.

### a- Transformation par Agrobacterium tumefaciens de plantes de maïs par un construit antisens d'un gène de la voie de biosynthèse de la lignine : CCoAOMT.

La transformation du maïs par *Agrobacterium tumefaciens*, à été réalisée à l'aide d'un vecteur sous la forme d'un plasmide d'environ 50 kb obtenu par recombinaison dans *Agrobacterium* entre un vecteur pBIOS et un vecteur superbinaire (pSB1). Ce vecteur comporte notamment : une région d'origine de réplication plasmidique Col EI, nécessaire au maintien et à la multiplication du plasmide dans *Escherichia coli*, non fonctionnelle dans *Agrobacterium tumefaciens*, une origine de réplication fonctionnelle dans *Agrobacterium tumefaciens*, les régions virB, virC et virG supplémentaires d'*Agrobacterium tumefaciens* qui augmentent l'efficacité de transformation, les gènes de résistance à la tétracycline (Tétra) et à la spectinomycine (Spect) sous contrôle de promoteurs s'exprimant uniquement dans les bactéries,

On introduit dans ce vecteur un ADN de transfert (ADN-T) porteur de deux cassettes d'expression. La première cassette comprend : la séquence promotrice du CsVMV (WO 97/48819), une séquence issue de la séquence CCoAOMT du maïs en orientation antisens (CCoAOMT1 de maïs Numéro d'accession GenBank AJ242980, SEQ ID N° 7, nucléotides 1 à 1143) et la séquence terminateur NOS (nopaline synthase). La seconde cassette comprend : la séquence promotrice du gène d'actine de riz et la séquence du premier intron de ce gène, un gène de sélection à un herbicide, et une séquence terminateur NOS.

La transformation a été effectuée via le protocole de transformation par *Agrobacterium tumefaciens* d'Ishida et al. (Nature Biotechnology, 14, 745-750, 1996).

Des épis immatures d'une lignée produite en serre, sont prélevés 10 jours après la pollinisation et stérilisés pendant 15 minutes. Les embryons sont prélevés et mis en contact 5 minutes avec une suspension d'*Agrobacterium* contenant le vecteur décrit ci-dessus. Après avoir été retirés de la suspension d'*Agrobacterium*, les embryons sont mis en culture sur un milieu ne contenant ni de bactériostatique, ni d'agent sélectif. Cette co-culture a lieu durant 4 à 7 jours à l'obscurité. Après la co-culture, les embryons sont repiqués sur un milieu de callogénèse frais contenant le bactériostatique et l'agent sélectif. Un cal s'initie et se développe à partir des cellules transformées de ces embryons. L'étape de callogénèse se passe à 25 °C à l'obscurité et dure 5 semaines. Les embryons-cals sont repiqués sur milieu frais toutes les 2 à 3 semaines. Au terme de cette étape les cals sont repiqués sur un milieu de régénération pendant 5 semaines avec repiquage du cal sur milieu frais après 2 à 3 semaines. On obtient une régénération de plantules à partir des cals, qui sont mises en enracinement en tube, une fois qu'elles sont assez développées. Après 10-15 jours en tube, les plantules sont acclimatées en phytotron avant d'être transférées en serre. Les transformants sont ensuite cultivés et croisés avec du pollen d'une plante non transgénique pour produire la génération T1.

### b- Transformation par Agrobacterium tumefaciens de plantes de maïs par un construit RNAi d'un gène de la voie de biosynthèse de la lignine : CCoAOMT.

Construction d'un vecteur comprenant un fragment de la séquence d'un gène de la voie de biosynthèse de la lignine CCoAOMT en orientation sens et antisens.

Ce vecteur a été construit en utilisant le fragment BglII de 768bp issu de la séquence CCoAOMT1 de maïs (numéro d'accession AJ242980, SEQ ID N° 7 (fragment 334-1102)) grâce au système Gateway (Invitrogen). Ce vecteur porte une cassette d'expression constituée du promoteur CsVMV, du fragment de la séquence CCoAOMT1 en orientation antisens, du premier intron du gène de la tubuline de riz, du fragment de la séquence CCoAOMT1 en orientation sens et du terminateur NOS. Le fragment de la séquence CCoAOMT1 a été choisi pour permettre de contrôler l'expression des séquences CCoAOMT1 et CCoAOMT2.

Ce vecteur a été utilisé pour la transformation du maïs par biolistique, comme décrit ci-dessous.

La méthode de transformation par biolistique implique la co-transformation des cellules végétales d'une part avec le gène d'intérêt, et d'autre part avec un plasmide porteur d'une cassette d'expression comprenant un gène de sélection (par exemple, un gène de résistance à un herbicide).

Des embryons immatures prélevés 10 jours après la pollinisation sont mis en culture sur un milieu osmotique pendant 4 jours puis bombardés avec des particules d'or enrobées des plasmides contenant le gène d'intérêt et le gène de sélection. Les embryons sont ensuite repiqués sur un milieu de callogénèse, puis les cals développés sont placés sur un milieu de régénération, et les plantes régénérées comme vu dans l'exemple 2.a.

### c- Analyses phénotypiques des transformants anti-sens et RNAi

Le protocole d'inoculation artificielle par *Fusariom monoliforme*, écrit dans l'exemple 1 pour les lignées mutantes de maïs, est utilisé pour tester la tolérance des transformants anti sens et RNAi du gène CCoAOMT.

La méthodologie décrite dans cet exemple (points a-, b- ou c-) pour inhiber le gène CCoAOMT peut également être reproduite avec d'autres gènes, et notamment le gène CCR1.

### Exemple 3: Association génétique entre le gène CCR1, CCoAOMT1, CCoAOMT2, C4H et la tolérance à la fusariose chez le maïs.

Afin de comparer l'effet potentiel de différents allèles du gène CCR1 chez le maïs, on réalise des études d'association.

Ces études d'association également appelées analyse de déséquilibre de liaison permettent d'associer un allèle donné avec un phénotype particulier. Le terme « déséquilibre de liaison » se réfère à l'association non-aléatoire entre deux allèles pris à des loci différents. Ainsi, il y a un déséquilibre de liaison entre deux allèles (1 et 2) si l'allèle 1 présent à un locus donné a tendance à être présent chez les mêmes lignées que l'allèle 2 à un autre locus. Le déséquilibre de liaison peut être favorisé par plusieurs phénomènes comme la liaison génétique, la sélection, la dérive génétique, la migration ou le mélange des populations. Cette méthode a fait l'objet d'une revue chez les plantes (Flint-Garcia et al., (2003). Rev. Plant. Biol, 54: 357-74).

On recherche des associations entre un marqueur moléculaire spécifique du gène CCR1 et le caractère de tolérance à la fusariose en tenant compte de la structuration résiduelle du panel utilisé.

Des amorces spécifiques du gène CCR1 sont développées. On identifie ensuite les différents allèles existants dans le panel, soit en séquençant les produits PCR sur l'ensemble des lignées, soit en recherchant les polymorphismes nucléotidiques (SNPs) à une ou plusieurs positions particulières sur l'ensemble des lignées. En théorie, il est préférable d'étudier en priorité les SNP qui affectent la fonction de la protéine ou son expression (non synonymes ou mutations non sens) car la probabilité de faire varier le phénotype est plus importante. Cependant, même si le polymorphisme détecté n'induit pas de changement sur la séquence en acide aminé de la protéine, il peut affecter quand même la fonction du gène en altérant la régulation de l'expression, la stabilité, l'épissage, ou la localisation des ARNm.

En langage statistique, une association est effectuée par une analyse de variance : la donnée quantitative expliqué est le phénotype observé (Y) et le facteur qualitatif variable est le marqueur de polymorphisme (X). L'analyse de variance nécessite que l'ensemble des données suivent un modèle linéaire de type Y = Xθ +e où e correspond à l'erreur expérimentale. L'analyse de variance permet ensuite d'estimer les moyennes théoriques de chaque facteur et aussi les différences entre ces moyennes, ce qui détermine l'existence ou l'absence d'une association statistique entre le polymorphisme et le caractère.

Les tests d'association permettent ainsi de déterminer l'existence d'une corrélation entre un polymorphisme nucléotidique (SNP) et la résistance à la fusariose.

Selon le principe rappelé ci-dessus, avec le gène CCR1, des études d'association sont réalisés sur le maïs en utilisant un panel de lignées d'environ 350 lignées sélectionnées pour représenter la variabilité génétique du maïs mais aussi choisies pour leur aptitude à la sensibilité ou tolérance à la fusariose épis. Une année d'expérimentation sur deux lieux pendant une année a été réalisée sur ce panel. Chaque lignée a été inoculée avec une suspension de spore de *Fusarium monoliforme* dans le canal des soies, 1-5 jours après floraison femelle. Les symptômes de sensibilité à la fusariose ont été notés sur les spathes (note de 1-9) et sur épis à maturité (note de 1-7) pour chaque lignée. On peut identifier des SNPs pour les gènes suivants, qui font partie de la voie de biosynthèse des phénylpropanoïdes et de la lignine :
- C4H : Cinnamic acid 4-hydroxylase (EC 1.14.13.11).
- CAD : Cinnamyl-alcohol dehydrogenase (EC 1.1.1.195) (CAD) (Brown-midrib 1protein).
- PAL : Inducible phenylalanine ammonia-lyase Fragment).
- CCR : Putative cinnamoyl-CoA reductase.
- 4Cl3: 4-coumarate--CoA ligase 4CL3 (EC 6.2.1.12).
- CCoAOMT1: Caffeoyl-CoA O-methyltransferase 1 (CCoAOMT-1).
- CCoAOMT2: Caffeoyl-CoA O-methyltransferase 2 (EC 2.1.1.104) (Trans-caffeoyl-CoA3-O-methyltransferase 2) (CCoAMT-2).
- CCR1 : cinnamoyl CoA reductase 1
- COMT : Caffeic acid 3-O-methyltransferase (EC 2.1.1.68) (S-adenosysl-L-methionine:caffeic acid 3-O-methyltransferase) (COMT ou CAOMT).

Ces SNPs peuvent aisément être identifiés sur des bases de données tels la base TIGR (The Institute for Genomic Research) ou GenBank.

Pour chaque gène, une sélection de SNPs (entre 1-5 SNP) représentant au moins 95% de la variabilité allélique est utilisé pour réaliser le génotypage des 350 lignées du panel. Comme décrit ci-dessus, des tests d'association sont réalisés de façon à déterminer l'existence d'une corrélation entre un polymorphisme nucléotidique (SNP) et la résistance à la fusariose.

Ainsi, on a pu mettre en évidence une association significative au seuil de 5% entre le polymorphisme du gène CCoAOMT2 et la variation de la fusariose épis (moyennes ajustées sur les 2 lieux d'essais), le gène CCoAOMT2 expliquant 8% de la variance phénotypique. Ces résultats démontrent l'implication du gène CCoAOMT2 dans le mécanisme de résistance / tolérance à la fusariose.

### Exemple 5 : Recherche d'allèles nouveaux pour le gène CCR1 dans les ressources génétiques ou dans une collection de mutations induites par un agent chimique chez le maïs

On utilise une collection représentative appelée ci-après « core collection » présentant une variabilité moléculaire maximale en relation avec des critères d'origines à la fois géographiques et temporels, afin d'identifier de nouveaux allèles du gène CCR1 par la méthode dite d'EcoTilling décrite dans la littérature (Mejlhede et al. 2006, Plant Breeding, 125 : 461-467).

Simultanément au criblage de la « core collection », une recherche « d'allèles induits » est réalisé à partir d'une collection de maïs issue de la réunion de collections de maïs muté par des radiations ionisantes ou un agent chimique (EMS). Cette collection de maïs permet d'identifier des allèles non présents naturellement dans les variétés de maïs existantes naturellement. De plus, 5% des mutations découvertes sont des allèles nuls du gènes CCR1, c'est-à-dire un allèle du gène CCR1 où la fonction est annulée (absence d'expression). Cette méthode dite de TILLING (Targeting Induced Local Lésions IN Genomes) est décrite dans la littérature (McCallum et al. 2000, Nature Biotechnology. 18 : 455-457).

Les allèles issus des approches de Tilling ou d'EcoTilling sont identifiés grâce à la technologie développée par Henikoff et Comaï (Plant J. 2006 Feb;45(4):684-94, Annu Rev Plant Biol. 2003;54:375-401). Pour cela, des amorces spécifiques du gène CCR1 sont développées. Le produit PCR correspondant à l'amplification d'une copie du gène CCR1 est ensuite comparée avec un même produit PCR issu d'un ADN issue d'une variété de référence. La méthode d'analyse des mutations est réalisée par identification d'hétéroduplex d'ADN créés par la renaturation des brins d'ADN provenant de la copie de la variété testée et la variété de référence. En cas de présence d'une mutation dans la variété testée, il s'ensuit une anomalie d'appareillement (ou « mismatch »). Cette anomalie est détectée puis coupée par une enzyme de type endonucléase reconnaissant cette structure particulière (par exemple l'enzyme Cell ou l'enzyme EndoI). La reconnaissance du mismatch est ensuite détectée par électrophorèse. Par exemple sur gel d'agarose, sur séquenceur automatique en plaque (type LICOR), sur séquenceur capillaire sur ABI3100 ou sur ABI3730.

Chaque différence détectée constitue un « haplotype » particulier du gène CCR1. L'intérêt de chaque haplotype est ensuite évalué par une évaluation phénotypique.

L'ensemble des variétés présentant des haplotypes distincts (provenant soit d'un criblage des ressources génétiques soit de la découverte de mutations induites artificiellement) sont croisés avec une variété de référence afin de disposer d'une collection d'allèles de CCR1 dans un même fond génétique élite.

Des évaluations phénotypiques sont entreprises pour déterminer si la présence d'un haplotype particulier est corrélée à une résistance à la fusariose. Ce même type d'expérimentation est étendu à d'autres types de pathogènes du maïs. Une note de sensibilité est ensuite établie pour chaque variété afin d'établir la corrélation entre la présence d'un haplotype ou mutation particulière et une note de résistance à la fusariose.

### Exemple 6 : Utilisation des allèles identifiés en sélection variétale

Les haplotypes conférant le plus fort niveau de résistance sont ensuite utilisés en sélection variétale. Le brassage génétique entre la variété résistante et des variétés présentant d'autres qualités agronomiques telles que des rendements élevés, de fort taux en protéines, une aptitude à la résistance à la germination sur pied, permet de sélectionner de nouvelles variétés regroupant l'ensemble des caractères souhaités. L'allèle conférant la résistance à la fusariose est suivi par marquage moléculaire au cours de ce processus de sélection variétale.

Ainsi les allèles identifiés par Tilling, EcoTilling ou validés par test d'association, permettent de proposer des plans de croisements entre les individus les plus complémentaires sur la base de leurs allèles (ici un ou plusieurs allèles conférant une résistance à la fusariose) puis d'utiliser les connaissances accumulées pour proposer les croisements pertinents des parents potentiels afin de pyramider des gènes (accumuler différents haploytypes) devant aboutir à l'obtention de variétés améliorées.

## Revendications

1. Méthode pour augmenter la tolérance à un pathogène fongique chez le maïs comprenant une étape consistant à altérer qualitativement et/ou quantitativement la synthèse de lignines, par l'inhibition totale ou partielle de l'expression d'un gène codant pour la cinnamoyl CoA reductase (CCR) impliqué dans la voie de biosynthèse de la lignine, la tolérance audit pathogène fongique étant augmentée par rapport à un maïs non altéré.

2. Méthode selon la revendication 1, dans laquelle le pathogène fongique est du genre *Fusarium*.

3. Méthode selon la revendication 1 ou 2, dans laquelle le gène de la voie de biosynthèse de la lignine est CCR1, dont la séquence d'un allèle représentatif est SEQ ID N° 1.

4. Méthode selon l'une des revendications 1 à 3, dans laquelle l'inhibition est obtenue par mutation dudit gène par insertion d'un élément transposable ou d'un T-DNA ou par mutagénèse physique (EMS, Rayons X, UV).

5. Méthode selon l'une des revendications 1 à 3, dans laquelle l'inhibition est obtenue par transformation dudit maïs par un construit en anti-sens ou sur expression, ou RNAi.

## Patentansprüche

1. Verfahren zum Erhöhen der Toleranz gegenüber einem pilzlichen Pathogen beim Mais, umfassend einen Schritt, der daraus besteht, dass man durch vollständige oder teilweise Hemmung der Expression eines Gens, das für die Cinnamoyl-CoA-Reduktase (CCR), die an dem Ligninbiosyntheseweg beteiligt ist, codiert, die Synthese von Ligninen qualitativ und/oder quantitativ verändert, wobei die Toleranz gegenüber dem pilzlichen Pathogen im Vergleich zu einem unveränderten Mais erhöht ist.

2. Verfahren nach Anspruch 1, wobei das pilzliche Pathogen zur Gattung *Fusarium* gehört.

3. Verfahren nach Anspruch 1 oder 2, wobei es sich bei dem Gen des Ligninbiosynthesewegs um CCR1 handelt, bei dem die Sequenz eines repräsentativen Allels SEQ ID NO: 1 ist.

4. Verfahren nach einem der Ansprüche 1 bis 3, wobei die Hemmung durch Mutation des Gens mittels Insertion eines transponierbaren Elements oder einer T-DNA oder mittels physikalischer Mutagenese (EMS, Röntgenstrahlen, UV) erzielt wird.

5. Verfahren nach einem der Ansprüche 1 bis 3, wobei die Hemmung durch Transformation des Maises mittels eines Antisense-Konstrukts oder bei der Expression, oder mittels RNAi erzielt wird.

## Claims

1. Method for increasing tolerance to a fungal pathogen in maize comprising a step consisting in qualitatively and/or quantitatively altering the synthesis of lignins, by total or partial inhibition of the expression of a gene coding for the cinnamoyl CoA reductase (CCR) involved in the lignin biosynthesis route, the tolerance to said fungal pathogen being increased relative to a non-modified maize.

2. Method according to Claim 1, **characterized in that** the fungal pathogen is of the *Fusarium* genus.

3. Method according to Claim 1 or 2, **characterized in that** the lignin biosynthesis route gene is CCR1, the sequence of a representative allele whereof is SEQ ID No.1.

4. Method according to one of Claims 1 to 3, **characterized in that** the inhibition is achieved by mutation of said gene by insertion of a transposable element or of a T-DNA or by physical mutagenesis (EMS, X-rays, UV).

5. Method according to one of Claims 1 to 3, **characterized in that** the inhibition is achieved by transformation of said maize by an antisense construct, or overexpression, or RNAi.


### SEQUENCE LISTING

<110> Biogemma
<120> MAIS PRESENTANT UNE TOLERANCE ACCRUE AUX MALADIES FONGIQUES
<130> BGM 40 - WO
<150> FR 0609295
   <151> 2006-10-24
<160> 7
<170> PatentIn version 3.3
<210> 1
   <211> 1116
   <212> DNA
   <213> Zea mays
<400> 1
   atgaccgtcg tcgacgccgt cgtctcctcc accgatgccg gcgcccctgc cgccgccgcc      60
   gcaccggtac cggcggggaa cgggcagacc gtgtgcgtca ccggcgcggc cgggtacatc     120
   gcctcgtggt tggtgaagct gctgctcgag aagggataca ctgtgaaggg caccgtgagg     180
   aacccagatg acccgaagaa cgcgcacctc agggcgctgg acggcgccgc cgagcggctg     240
   atcctctgca aggccgatct gctggactac gacgccatct gccgcgccgt gcagggctgc     300
   cagggcgtct tccacaccgc ctcccccgtc accgacgacc cggagcaaat ggtggagccg     360
   gcggtgcgcg gcaccgagta cgtgatcaac gcggcggcgg aggccggcac ggtgcggcgg     420
   gtggtgttca cgtcgtccat cggcgccgtg accatggacc ccaagcgcgg gcccgacgtc     480
   gtggtcgacg agtcgtgctg gagcgacctc gagttctgcg agaaaaccag gaactggtac     540
   tgctacggca aggcggtggc ggagcaggcg gcgtgggagg cggcccggcg gcggggcgtg     600
   gacctggtgg tggtgaaccc cgtgctggtg gtgggccccc tgctgcaggc gacggtgaac     660
   gccagcatcg cgcacatcct caagtacctg gacggctcgg cccgcacctt cgccaacgcc     720
   gtgcaggcgt acgtggacgt gcgcgacgtg gccgacgcgc acctccgcgt cttcgagagc     780
   ccccgcgcgt ccggccgcca cctctgcgcc gagcgcgtcc tccaccgcga ggacgtcgtc     840
   cgcatcctcg ccaagctctt ccccgagtac cccgtcccag ccaggtgctc cgacgaggtg     900
   aatccgcgga agcagccgta caagttctcc aaccagaagc tccgggacct ggggctgcag     960
   ttccggccgg tcagccagtc gctttacgac acggtgaaga acctccagga gaagggccac    1020
   ctgccggtgc tcggagagcg gacgacgacg gaggccgccg acaaggatgc ccccgcggcc   1080
   gagatgcagc agggagggat cgccatccgt gcctga                              1116
<210> 2
   <211> 1116
   <212> DNA
   <213> Zea mays
<400> 2
   atgaccgtcg tcgacgccgt cgtctcctcc accgatgccg gcgcccctgc tgccgccgcc      60
   accgcggtac cggcggggaa cgggcagacc gtgtgcgtga ccggcgcggc cgggtacatc     120
   gcctcgtggt tggtgaagct gctgctcgag aagggataca ctgtgaaggg caccgtcagg     180
   aacccagatg acccgaagaa cgcgcacctc aaggcgctgg acggcgccgc cgagcggctg     240
   atcctctgca aggccgatct gctggactac gacgccatct gccgcgccgt gcagggctgc     300
   cagggcgtct tccacaccgc ctcccccgtc accgacgacc cggagcaaat ggtggagccg     360
   gcggtgcgcg gcaccgagta cgtgatcaac gcggcggcgg aggccggcac ggtgcggcgg     420
   gtggtgttca cgtcgtccat cggcgccgtg accatggacc ccaagcgcgg gcccgacgtc     480
   gtggtcgacg agtcgtgctg gagcgacctc gagttctgcg agaaaaccag gaactggtac     540
   tgctacggca aggcggtggc ggagcacgcg gcgtgggaga cggcccggcg gcggggcgtg     600
   gacctggtgg tggtgaaccc cgtgctggtg gtgggccccc tgctgcaggc gacggtgaac     660
   gccagcatcg cgcacatcct caagtacctg gacggctcgg cccgcacctt cgccaacgcc     720
   gtgcaggcgt acgtggacgt gcgcgacgtg gccgacgcgc acctccgcgt cttcgagagc     780
   ccccgcgcgt ccggccgcca cctctgcgcc gagcgcgtcc tccaccgcga ggacgtcgtc     840
   cgcatcctcg ccaagctctt ccccgagtac cccgtcccag ccaggtgctc cgacgaggtg     900
   aatccgcgga agcagccgta caagttctcc aaccagaagc tccgggacct ggggctgcag     960
   ttccggccgg tcagccagtc gctttacgac acggtgaaga acctccagga gaagggacac    1020
   ctgccggtgc tcggagagcg gacgacgacg gaggccgccg acaaggatgc ccccacggcc    1080
   gagatgcagc agggagggat cgccatccgt gcctga                              1116
<210> 3
   <211> 1197
   <212> DNA
   <213> Zea mays
<400> 3
   agcagtagta atagtaggag tatattgtaa aattgaggag cattactgta ttaccgatct      60
   cgtgatttac gtatcgaaac ggcggcgaca attcccaaga aggaagagga ggagaaagat     120
   tacaggggca agaaagagcc aaaaaaaaag ccggtggata attaatgctg cgggtcgtct     180
   attatgattg gaatgaggat gcggccggcg gcgccgtcgg tggcggtggt ggggcaggcc     240
   aggccaggcc atgttttcac ttgacgcggc ggcagagggt gacgccgtcg ccgacgggga     300
   gctggcagat ctcgacgcgg tcgtcggcgg cgagcgcctt gttgaggacg agcacgaagt     360
   cgcggtagaa gcggatgtac ttgcgcatgg gcgcgtcgtc ggggagcacg acggagccgt    420
   tccacagcgt gttgtcgtag ccgatgaggc cgcccagctt caccagcttc agcagccgct    480
   cgtggtagtt gaggtagttg tccttgtcgg cgtccacgaa gacgaagtcg aacgacccgt    540
   ggttcttctc ctccgcgatg aggtcgtcga ggacggggag cgcgggaccc tcgcggaagt    600
   cgatcttgtg ggcgacgccg gccttctcga tgcagggcag gcccagctcg tagttctcgc    660
   ggttgatgtc catggccaag atcgtgccgt cctccgggag ggcgagcgcc gtggcgagga    720
   gggagtagcc ggtgtagacg ccgatctcca tggtcttctt ggcgccgatg agcttgatga    780
   gcatgttcag gaactgcccc tcgtcggcgg aggtcgtcat caggttccat gggtgcttgg    840
   cggtgatctc gcggagctcc ttcatgctct ccggctcccg cgggtacacg ctcgtgtcca    900
   ggatgtactg gtagaggtcg tcgctcttga gcaggctctt gtggccgacc tcggagtgcc    960
   gcgtcttctg ctcgccgttg ccgttggcct gctgctcctg cgccggcgcc gcctcggtcg   1020
   ccgtggtggc cattgcgtgc agtgtagtta gctgaacgaa cgagtccctc ctggagatct   1080
   ggggtgcggc aatagaacta gctagcgcgt cgggtactcg ggtattgctg gattgaccga   1140
   ccttgcctgc cggggcggct tatataacgc gcggcggcaa ggcgcggacg cgtgggt      1197
<210> 4
   <211> 1195
   <212> DNA
   <213> Zea mays
<220>
   <221> misc_feature
   <222> (1148)..(1170)
   <223> n is a, c, g, or t
<400> 4
   cgcaagccag tgccgcgccc agatctccgc gacagatcag tcgttcgtcc agctaactgc     60
   actgcacgca atggccacca cggcgaccga ggcgaccaag acgactgcac cggcgcagga    120
   gcagcaggcc aacggcaacg gcaacggcaa cggcgagcag aagacgcgcc actccgaggt    180
   cggccacaag agcctgctca agagcgacga cctctaccag tacatcctgg acacgagcgt    240
   gtacccgcgg gagccggaga gcatgaagga gctgcgcgag atcaccgcca agcacccatg    300
   gaacctgatg accacctccg ccgacgaggg ccagttcctc aacatgctca tcaagctcat    360
   cggcgccaag aagaccatgg agatcggcgt ctacaccggc tactcgctcc tcgccaccgc    420
   gctcgcactc ccggaggacg gcacgatctt ggccatggac atcaaccgcg agaactacga    480
   gctaggcctt ccctgcatca acaaggccgg cgtgggccac aagatcgact tccgcgaggg    540
   ccccgcgctc cccgtcctgg acgacctcgt ggcggacaag gagcagcacg ggtcgttcga    600
   cttcgccttc gtggacgccg acaaggacaa ctacctcagc taccacgagc ggctcctgaa    660
   gctggtgagg cccggcggcc tcatcggcta cgacaacacg ctgtggaacg gctccgtcgt    720
   gctccccgac gacgcgccca tgcgcaagta catccgcttc taccgcgact tcgtcctcgc    780
   cctcaacagc gcgctcgccg ccgacgaccg cgtcgagatc tgccagctcc ccgtcggcga    840
   cggcgtcacg ctctgccgcc gcgtcaagtg aaaaaaagaa gaagaagaaa aaaaacataa    900
   taccctgcgt tcctgctgcc ccggctgtct ggcccccact actgccaccg acggcggcgc    960
   cgcacccccg ttccaatcat atcgtagacg acgcgcagca ttaaattatc aatcaccggc   1020
   tctggctctt tcttggccct gtactgtact attaatgttc cgttcttgtt tttttattcg   1080
   gaattgtcgc cgtttcagta tacgtaaatc tcgaggtcga taatacagta atactaccaa   1140
   tttaactnnn nnnnnnnnnn nnnnnnnnnn gtcgacgcgg ccgcgaattc ggatc        1195
<210> 5
   <211> 1719
   <212> DNA
   <213> Zea mays
<220>
   <221> misc_feature
   <222> (156)..(177)
   <223> n is a, c, g, or t
<400> 5
   acgacacaaa cacacacccc acctaccccg gccggaccgg caggcagcac agcatggacc     60
   tcgccctcct agagaaggcc ctgctgggcc tgttcgccgc ggctgtggtg gccatcgccg    120
   tggccaagct gaccggcaag cggtaccgcc tcccannnnn nnnnnnnnnn nnnnnnntgg    180
   tgggaaactg gctgcaggtg ggcgacgacc tgaaccaccg caacctgatg gccatggcga    240
   agcggttcgg cgacatcttc ctgctgcgca tgggcgtgcg caacctggtg gtggtgtcga    300
   ccccggagct ggccaaggag gtgctccaca cgcagggcgt ggagttcggc tcccgcaccc    360
   gcaacgtggt gttcgacatc ttcacgggca aggggcagga catggtgttc acggtgtacg    420
   gcgaccactg gcgcaagatg cggcgcatca tgaccgtccc cttcttcacc aacaaggtgg    480
   tggcccagaa ccgcgccggg tgggaggagg aggcccggct ggtggtggag gacgtgagga    540
   aggaccccga ggccgcggcc ggcggcgtcg tgctccgccg ccgcctccag ctgatgatgt    600
   acaacgacat gttccgcatc atgttcgacc gccggttcga cagcgagcac gacccgctct    660
   tcaacaagct caaggcgctc aacgcggagc gcagccgcct gtcgcagagc ttcgagtaca    720
   actacggcga cttcatcccc gtgctccgcc ccttcctccg cggctacctc aaccgctgcc    780
   acgacctcaa gacgcgccgc atgaaggtct tcgaggacaa cttcgtacag gagcgcaaga    840
   aggtgatggc tcagactggt gagatccggt gcgccatgga tcacatcctc gaggccgaga    900
   ggaagggcga gatcaaccac gacaacgtcc tctacatcgt cgagaacatc aacgtcgcag    960
   cgatcgagac gacactgtgg tcgatcgagt ggggcatcgc cgagctggtg aaccacccgg   1020
   ccatccagca caagctccgg gaggagctcg cctcggtgct gggcgccggc gtgcctgtga   1080
   cggagccgga cctcgagcgc ctcccctacc ttcaggccat cgtcaaggag acgctccgcc   1140
   tgcgcatggc catcccgctg ctggtccccc acatgaacct caacgacggc aagctcgccg   1200
   gctacgacat ccccgccgag tccaagatcc tcgtcaatgc ctggttcctc gccaacgacc   1260
   ccaagaggtg ggtgcggccc gacgagttcc ggcccgagcg cttcctggag gaggagaagt   1320
   ccgtggaggc ccacggcaac gacttccgct tcgtgccctt tggggtcggc cgccggagct   1380
   gccctgggat catcctcgcg ctgcctatca tcggcatcac cctgggccgg ctggtgcaga   1440
   acttccagct gctgccgccg ccggggctgg acaagatcga caccacggag aagcccggcc   1500
   agttcagcaa ccagatcgcc aagcatgcca ccatcgtctg caagcccctc gaggcctaga   1560
   aatcaatgcc tgtttcctgc acgcgccccc gcagatgaag cactatgtat tttgtctttt   1620
   ttttgtgtgt tgtgtttttt ttactaagag gagatgtatt tcttgttcgt aaaatgcact   1680
   tagtcaaatg gatcgagatt atgttgatca ttaaacccc                           1719
<210> 6
   <211> 2131
   <212> DNA
   <213> Zea mays
<220>
   <221> misc_feature
   <222> (31)..(41)
   <223> n is a, c, g, or t
<220>
   <221> misc_feature
   <222> (1905)..(1967)
   <223> n is a, c, g, or t
<400> 6
   taaataggac cgggcccccc cccgagtctg nnnnnnnnnn ntatgaaaca cggtactccg     60
   tagctgtatt gccgtaatca ggcttgttct ttgggtcaaa aacaacatag agacgacgtc    120
   tatatcgttt atgaacatta cacacagggc caacaaacag acaaactaaa acctctttct    180
   ggcaacatgt gttccacagg aatacgtgtc ccttctgttg ccagttcatg aatagtagtg    240
   ttaccgccat tacatcgcct tttttttcgc agcagcgtgg tggccgtccg agaagaagct    300
   gcatacgggc ctcagtgaac accggcggcg agcctggctc tcaagtcctt cctcaggatc    360
   ttgcccgacg ggttcttggg gatggattcg gtgaagaaga ccttgtggat cttcttgtag    420
   aaaaccacct ccttggcgac gaattgcttg atctcatcct cggtgacttg agaaccttcg    480
   gtccgcacga tgaaggcgac cgggatttca ccagcaaggt catcgttcat tgatacgacg    540
   gcggcgtcct tgatctccgg gtgcgtgatg aggagcgcct ccagctccgc cggcggcacc    600
   tggaagccct tgtacttgat gatctccttg agcctgtcga cgatgaagat ctcgtcgtcg    660
   tcgtccacgt agccgatgtc tccggtgtgc agccagccgt ccttgtcgat ggtgttcttc    720
   gtcgactcgg ggtcgttcag gtaacctttc atgatctgct ccccgcggat gcagatctcg    780
   ccgggctggt tccggccgag ggcggcgccg gtgtcggggt cgacgatctt cagctccgcg    840
   ttccgcacca cggtgccgca cgacccggac ttgaccgggt acggctcctt ggcgaaggcc    900
   aggcacatcg ccagcacggg gcctgcctcc gtcatcccgt acccctgccc gagcacggca    960
   ttggggatct tggccatgaa ggcgtcctgg agctccttgc ccatgggcgc ggcgccggac   1020
   atgaccatgc ggatggacgc gaggtcgccg gcggtcacgc gggggctctt ggcgatctcc   1080
   accacgatgg gcggcacgaa gggcgcgatg gtgatcacgt acctgcgcac caggtcaacc   1140
   agcgcgccca ggtcgaactt gcgcatgatc acgatggtgg agcccgcgcg caggccggcc   1200
   agcagcaccg agttcagcga gtagatgtgg aacagcggca gcaggcacag caccacgtcg   1260
   tccttgcgga agtacaggtt cgggttctcg ccatcaacct gctgcgcgac gctggtgatg   1320
   aggctgcggt gggtgagcat gacgcccttg ggcagcccgg tggtgccgga ggagtagggc   1380
   agcgcgacga cgtcgtcggg gtggatgtcg gcgtcagcct ccagctcctc ggccgcgatc   1440
   agctcggcga actccacgca gccgtcgaag cgcccgtcga cggtgaccac ggggaatgcc   1500
   cccgctccgc cgcgaactcc cgcaccttct tcaacggcgc aggcctcggt cacgatgagc   1560
   cgggcgccgg ccgcctccgc ctggcggtgc acctcgtgcg gggtgtagaa cgggttggcc   1620
   gtggtggtgg cggcgcccag gcgggcggcg cccaggaagg tgaaggcgaa ctcggggcag   1680
   ttgcggagca ggctcatcac cacgtcgccc ttgcccaccc ccatggcgcg cagccccgac   1740
   gcggcgcgcc gggacaggga ctccacctcc gcgtacgtgt acgacgcgcc cgtcagcccg   1800
   tcgatcaggc acgcccgctc cgccacctcg cccatcttcc cgaagcagta ggtgtgcagc   1860
   gccatgctgc tgtcgatctc gatgtcgggg agcttggacc ggaannnnnn nnnnnnnnnn   1920
   nnnnnnnnnn nnnnnnnnnn nnnnnnnnnn nnnnnnnnnn nnnnnnngtc tacggaaccc   1980
   atctcagacc tttgctcgct gccggatgga ctggcttgtc ggatgtcggg caggtaacgt   2040
   tgcgctgggc agctggttgg atggtagtat aggaaggaag gaaggagacg atggctggtg   2100
   cggtgcgacc tcgccggggc cggacgcgtg g                                  2131
<210> 7
   <211> 1136
   <212> DNA
   <213> Zea mays
<400> 7
   ctcgtgcccc aacgcgctag ctagttctat tgccgcaccc cagatctcca ggagggactc      60
   gttctttcag ctaactacac tgcacgcaat ggccaccacg gcgaccgagg cggcgccggc     120
   gcaggagcag caggccaacg gcaacggcga gcagaagacg cggcactccg aggtcggcca     180
   caagagcctg ctcaagagcg acgacctcta ccagtacatc ctggacacga gcgtgtaccc     240
   gcgggagccg gagagcatga aggagctccg cgaggtcacc gccaagcacc catggaacct     300
   gatgacgacc tccgccgacg aggggcagtt cctgaacatg ctcatcaagc tcatcggcgc     360
   caagaagacc atggagatcg gcgtctacac cggctactcc ctcctcgcca cggcgctcgc     420
   cctcccggag gacggcacga tcttggccat ggacatcaac cgcgagaact acgagctggg     480
   cctgccctgc atcgagaagg ccggcgtcgc ccacaagatc gacttccgcg agggtcccgc     540
   gctccccgtc ctcgacgacc tcatcgcgga ggagaagaac cacgggtcgt tcgacttcgt     600
   cttcgtggac gccgacaagg acaactacct caactaccac gagcggctgc tgaagctggt     660
   gaagctgggc ggcctcatcg gctacgacaa cacgctgtgg aacggctccg tcgtgctccc     720
   cgacgacgcg cccatgcgca agtacatccg cttctaccgc gacttcgtgc tcgtcctcaa     780
   caaggcgctc gccgccgacg accgcgtcga gatctgccag ctccccgtcg gcgacggcgt     840
   caccctctgc cgccgcgtca agtgaaaaca tgccctggcc tggcctgccc caccaccgcc     900
   accgacggcg ccgccggccg catcctcatt ccaatcataa tagacgaccc gcagcattaa     960
   ttatccaccg gctttttttt ttggctcttt cttgcccctg ttatctttct cctcctcttc    1020
   ttcttgggaa ttgtcgctgc cgtttcgata cgtaaatcac gagatcggta atacagtaat    1080
   gctcctcaat tttacaatat actcctacta ttactactgc taaaaaaaaa aaaaaa        1136